# EUROPÄISCHE PATENTSCHRIFT

(11) **EP 0 457 075 B1**
(45) Veröffentlichungstag und Bekanntmachung des Hinweises auf die Patenterteilung: **07.02.1996**
(21) Anmeldenummer: 91106676.9
(22) Anmeldetag: 25.04.1991
(51) Int. Cl.: C07D 475/14, A61K 9/16

(54) **Verfahren zur Herstellung von rieselfähigen, nichtstaubenden bindemittelfreien Riboflavinsprühgranulaten oder -mikrogranulaten aus reinem Riboflavin**
Process for the preparation of dust and binderfree, free flowing spray granulates or microgranulates of riboflavin from pure riboflavin
Procédé de préparation de granulats ou microgranulats pour pulvérisation de riboflavine, non poussiéreux, sans liant et à écoulement facile à partir de riboflavine pure

(30) Priorität: 04.05.1990 DE 4014262
(43) Veröffentlichungstag der Anmeldung: 21.11.1991
(73) Patentinhaber: BASF Aktiengesellschaft, D-67063 Ludwigshafen (DE)
(72) Erfinder: Grimmer, Johannes, W-6700 Ludwigshafen (DE); Kiefer, Hans, Dr., W-6706 Wachenheim (DE); Martin, Christoph, Dr., W-6800 Mannheim 1 (DE)

(56) Entgegenhaltungen:
- EP-A- 0 219 276
- EP-A- 0 307 767
- EP-A- 0 345 717
- EP-A- 0 414 115
- US-A- 4 994 458
- US-A- 5 000 888

## Beschreibung

Die vorliegende Erfindung betrifft ein Verfahren zur Herstellung von rieselfähigen, nichtstaubenden bindemittelfreien Riboflavinsprühgranulaten oder -mikrogranulaten aus reinem Riboflavin ausgehend von feinteiligem reinem Riboflavin.

Riboflavin (Vitamin B2) wird vielfältig verwendet in der Lebensmittel- und Pharmaindustrie als wesentlicher oder auch nur als färbender Zusatz zu Nahrungsmitteln und Pharmaka. Es fällt bei seiner synthetischen Herstellung oder der biotechnischen Gewinnung teilweise in Form von sehr feinteiligen Pulvern, teilweise in Form von langen gelben Nadeln an. In beiden Formen weist das Riboflavin sehr schlechte Handlings- und Fließeigenschaften auf.

Das feinteilige Pulver z.B. stäubt beträchtlich, weist eine sehr geringe Schüttdichte auf (meist unter 0,2 g/ml), lädt sich leicht elektrostatisch auf, zeigt ein schlechtes Fließverhalten und läßt sich daher nur mit großen Problemen weiterverarbeiten. Ein weiterer gravierender Nachteil des feinteiligen Pulvers ist der, daß es sich praktisch nicht zur Herstellung von Tabletten eignet, deren Riboflavinanteil 25 Gew.-% übersteigt (vgl. v. Bühler, "Vademecum for Vitamin Formulations", Wissenschaftliche Verlagsgesellschaft, Stuttgart, Seiten 98 bis 99).

Auch das bei langsamem Kristallisieren in der Wärme erhältliche nadelförmige Riboflavin führt infolge seines schlechten Fließverhaltens sowie infolge von Staubentwicklung und Aufladungserscheinungen zu Problemen bei der Weiterverarbeitung, wie beispielsweise bei der Mehlvitaminierung oder der Tablettierung.

Um diese Probleme zu lösen, wurde das Riboflavin teilweise unter Zusatz von Granulierhilfsmitteln granuliert um ein Produkt zu erhalten, welches akzeptable Fließ- und Kompressionseigenschaften aufweist. So werden in der EP-A-02 19276 Vitamin enthaltende Granulate beschrieben, die 90 bis 99 % Vitamin und ein Bindemittel enthalten.

Obwohl sich diese Granulate verfahrenstechnisch gut für die Weiterverarbeitung eignen, sei es zur Direkttablettierung, zur Bereitung andere Riboflavin enthaltender Arzneimittelpräparate oder von Vitamin B2 enthaltenden Lebens- und Futtermitteln, bleibt es häufig unbefriedigend, daß sie nicht aus reinem Wirkstoff bestehen. Dies gilt insbesondere für Pharmaware, da die Pharmakopoe ein Riboflavin mit einem Mindestgehalt von 98 % an Riboflavin vorschreibt.

Um bindemittelfreies Riboflavin für die Pharmaindustrie zur Verfügung zu haben, wird Riboflavin gemäß dem Verfahren der EP-A-0 307 767 in einem Lösungsmittel gelöst und mit einem zweiten Lösungsmittel, in welchem Riboflavin nicht löslich ist, welches aber mit dem ersten Lösungsmittel mischbar ist, in Form von sphärulitischen Kristallen mit guten Handlingseigenschaften ausgefällt. Dieses Verfahren ist jedoch technisch schwierig durchführbar und erfordert einen hohen technischen Aufwand, da mit größeren Mengen an Lösungsmitteln gearbeitet wird und die anfallenden Lösungsmittelgemische wieder aufgearbeitet werden müssen.

Weiterhin war aus EP-A-0 414 115 bekannt, daß man das leicht staubende feinteilige Riboflavinpulver durch Anwendung hoher Drücke zu Strängen oder Bändern pressen kann, die dann in verarbeitbare Partikel zerkleinert werden können. Die erhaltenen Partikel bieten bei der Direkttablettierung große Vorteile, sind aber durch die Festigkeit der Partikel für andere Anwendungsbereiche nicht so gut geeignet.

Es war daher die Aufgabe der Erfindung ein Verfahren zu entwickeln, mit dessen Hilfe man auf technisch einfache Weise , aus feinteiligem reinem Riboflavin bindemittelfreie Riboflavingranulate mit guten anwendungstechnischen Eigenschaften herstellen kann, d.h. Granulate, die einerseits geringe Staubentwicklung, ein gutes Fließverhalten, ein möglichst großes Schüttgewicht und möglichst geringe elektrostatische Aufladung zeigen, aber andererseits bei der Weiterverarbeitung auch wieder auf einfache Weise feinst verteilt werden können.

Es wurde nun überraschenderweise gefunden, daß man bei der Sprühwirbelschichttrocknung einer wäßrigen oder Wasser enthaltenden Suspension von feinteiligem reinem Riboflavin ein Riboflavingranulat mit ausgezeichneten anwendungstechnischen Eigenschaften erhält.

Gegenstand der Erfindung ist ein Verfahren zur Herstellung von rieselfähigen, nichtstaubenden bindemittelfreien Riboflavinsprüh- oder -miktrogranulaten mit einem Partikelgrößenbereich von 50 bis 450 µm aus feinteiligem reinem Riboflavin, das dadurch gekennzeichnet ist, daß man eine wäßrige oder mindestens 10 Gew.-% Wasser enthaltende Suspension etwa 5 bis 30 Gew.-% an reinem feinteiligem Riboflavin
a) einer Sprühwirbelschichttrocknung
b) einer Einstoffdüsenzerstäubungstrocknung oder
c) einer Scheibenzerstäubungstrocknung
bei Temperaturen von 20 bis 100°C zuführt, ohne daß der Suspension Bindemittel zugesetzt werden.

Besonders vorteilhaft gestaltet sich das erfindungsgemäße Verfahren, wenn man die wäßrige oder Wasser enthaltende Suspension des reinen feinteiligen Riboflavins einer Sprühwirbelschichttrocknung zuführt.

Als Ausgangsmaterial für das erfindungsgemäße Verfahren wird feinteiliges reines Riboflavin verwendet, wie es nach dem Stand der Technik beispielsweise beim einfachen Sprühtrocknen einer wäßrigen Suspension von Riboflavin oder aber beim raschen Fällen aus angesäuerten wäßrigen Riboflavinlösungen bei Temperaturen unterhalb von etwa 50°C, vorzugsweise 20 bis 30°C, oder aber bei raschem Fällen und raschem Abkühlen von heißen wäßrigen Riboflavinlösungen bei einem pH-Wert zwischen 0,8 und 6,5, anfällt. Dieses feinteilige Riboflavin weist üblicherweise einen mittleren größten Teilchendurchmesser von etwa 0,1 bis 50 µm, vorzugsweise 10 bis 30 µm, und eine Schüttdichte von weniger als 0,2 g/ml auf.

Größerteiliges nadelförmiges Riboflavin, wie es beispielsweise bei der Reinigung von rohem Riboflavin gemäß DE-OS 3 421 714 durch langsames Ausfällen von Riboflavin aus sauren wäßrigen Riboflavinlösungen bei Temperaturen zwischen 90 und 100°C erhalten wird, ist in Form seiner Suspension nicht als Ausgangsmaterial für das erfindungsgemäße Verfahren geeignet. Bei langsamem Fällen bei Temperaturen oberhalb 50°C erhaltenes größerteiliges nadelförmiges Riboflavin kann jedoch durch Umfällen oder aber durch Naßvermahlen (z.B. in einer Kolloidmühle) in geeignetes feinteiliges Riboflavin überführt werden.

Als reines Riboflavin bezeichnen wir erfindungsgemäß Riboflavin mit einem Reinheitsgrad von 96 bis 100, vorzugsweise 98 bis 100 %, dem keines der üblichen Bindungsmittel oder Granulierhilfsmittel zugesetzt wurde.

Das feinteilige reine Riboflavin wird mit Vorteil in Form einer wäßrigen Suspension mit einem Gehalt an Riboflavin von 5 bis 30, vorzugsweise 15 bis 25 Gew.-% eingesetzt. Man kann aber auch eine Suspension von Riboflavin an einem nicht zu hoch siedenden Lösungsmittel einsetzen, wenn dieses Lösungsmittel Wasser enthält. Der Wassergehalt in der Suspension sollte dann mindestens etwa 10 Gew.-% betragen. Als Losungsmittel kommen insbesondere wassermischbare Lösungsmittel, wie beispielsweise C₁- bis C₄-Alkanole, in Betracht.

Im Gegensatz zu der bekannten Sprühtrocknung von Riboflavinlösungen oder -suspensionen, bei der diese üblicherweise mittels einer Zweistoffdüse in einen Trockenturm eingesprüht wird, wird bei der erfindungsgemäß angewendeten Sprühwirbelschichttrocknung die Suspension kontinuierlich oder diskontinuierlich in eine Wirbelschicht aus trockenem Reaktionsprodukt eingesprüht. Die Trockeneinrichtung ist mit geeigneten Vorrichtungen versehen, die es gestatten, eine bestimmte Partikelgrößenfraktion zu gewinnen und den Granulationsprozess aufrechtzuerhalten (vgl. K. Kröll, Trocknungstechnik, Band II "Trockner und Trocknungsverfahren", 2. Auflage, Springer-Verlag, Berlin, 1978, Seiten 221 bis 223). Mit Vorteil arbeitet man in einem Sprühtrockner mit integriertem Fließbett (Abkürzung: FSD = Fluidized Spray Dryer), wie er in Chem.-Ing.-Tech. 59 (1987) Nr. 2, Seiten 112-117, insbesondere Seite 115 beschrieben ist.

Zur Durchführung der erfindungsgemäßen Sprühwirbelschichttrocknung von Riboflavinsuspensionen geht man im allgemeinen so vor, daß man
a) Riboflavin in Form eines Trockenpulvers, eines Sprüh- oder Mikrogranulats in einem Wirbelschichttrockner in einem auf 20 bis 100°C, vorzugsweise 50 bis 90°C, insbesondere 60 bis 80°C, gehaltenen Wirbelbett vorlegt,
b) hierzu eine wäßrige oder Wasser enthaltende Suspension des feinteiligen Riboflavins nach Maßgabe der Trocknungsgeschwindigkeit in versprühter Form zugibt,
c) die Riboflavinpartikel nach geeigneter Verweilzeit aus dem Wirbelbett abzieht und durch eine geeignete Vorrichtung in Partikelfraktionen auftrennt,
d) die Partikelfraktion im Partikelgrößenbereich von etwa 50 bis 450 µm ausschleust und
e) die feinerteiligen Partikel und/oder die durch Vermahlen von größeren Partikeln erhaltenen feinteiligen Partikel in den Granulierungsprozeß zurückführt.

Die Sprühwirbelschichttrocknung kann diskontinuierlich und kontinuierlich durchgeführt werden. Mit besonderem Vorteil arbeitet man kontinuierlich.

Zur Durchführung des Verfahrens muß zunächst erst einmal aus Riboflavintrockenpulver, wie es dem Stand der Technik entspricht, ein solches Riboflavinprodukt hergestellt werden, mit dessen Hilfe man ein Wirbeibett erzeugen kann. Bei diskontinuierlicher Verfahrensführung kann man ein relativ feinteiliges Produkt im Wirbelbett vorlegen. Je nach der Verweilzeit der Partikel im Wirbelschichttrockner erhält man dann ein Trockenprodukt mit einem kleineren oder größeren Partikelgrößenbereich. Partikel im Größenbereich von etwa 50 bis 450 µm weisen die gewünschten Handlingseigenschaften auf und werden daher als Wertprodukt gewonnen. Kleinere Partikel sowie durch geeignetes Vermahlen von größeren Partikeln erhaltenes Riboflavinprodukt wird als Wirbelbettmaterial für weitere Chargen verwendet.

Zur Durchführung des kontinuierlichen Verfahrens wird die wäßrige oder Wasser enthaltende Suspension von feinteiligem Riboflavin kontinuierlich in ein Wirbelbett, bestehend aus einem Riboflavintrockenprodukt, eingesprüht. Die Geschwindigkeit des Einsprühens wird so eingestellt, daß die Wirbelschicht eine dem gewünschten Trocknungsgrad entsprechende Temperatur aufweist. Sie wird dementsprechend letztlich bestimmt durch die Differenz der Eintritts- und Austrittstemperatur des Wirbelgases.

Bei kontinuierlicher Verfahrensführung geht man nur beim erstmaligen Anfahren des Wirbelschichttrockners von feinteiligem Riboflavin in der Wirbelschicht aus. Danach erhält man ein Trockenprodukt von nahezu konstantem Partikelgrößenverhältnis. Hiervon wird kontinuierlich ein bestimmter Teil entnommen und in Partikelgrößenfraktionen aufgetrennt. Die Partikelfraktion im Partikelgrößenbereich von 50 bis 450 µm wird als Wertprodukt ausgeschleust und die feinteiligen Partikel und/oder die durch Vermahlen von größeren Partikeln erhaltenen feinteiligen Partikel werden zur Aufrechterhaltung des Granulationsprozesses kontinuierlich in das Wirbelbett zurückgeführt. Jeweils etwa die als Wertprodukt entnommene Menge Riboflavin wird kontinuierlich in Form der wäßrigen Suspension von feinteiligem Riboflavin in das Wirbelbett eingesprüht.

Das zur Bildung des Wirbelbettes verwendete Wirbelgas hat im allgemeinen eine Eintrittstemperatur von 60 bis 250, vorzugsweise 140 bis 185°C und eine Austrittstemperatur von 40 bis 140, vorzugsweise 60 bis 85°C, wodurch sich Temperaturen von etwa 20 bis 100, vorzugsweise 50 bis 90°C, insbesondere 60 bis 80°C, im Wirbelbett ergeben.

Bei der Durchführung der erfindungsgemäßen Wirbelschichttrocknung ergeben sich im allgemeinen - je nach Korngrößensteuerung - folgende Partikelgrößenfraktionen:
1. etwa 6 bis 30 % im Partikelgrößenbereich bis 100 µm
2. etwa 25 bis 85 % im Partikelgrößenbereich von 100 bis 300 µm
3. etwa 1 bis 70 % im Partikelgrößenbereich von 250 bis 400 µm.

Zur Durchführung der erfindungsgemäßen Einstoffdüsenzerstaubungstrocknung verwendet man im allgemeinen eine Einstoffhohlkegeldüse, wie sie beispielsweise von der Firma Delawan unter der Bezeichnung "SDX-Standard" angeboten wird. Aber auch Hohlkegeldüsen der Firmen Lechler, Schlick und Lurgi sowie Hohlkegeldüsen gemäß EP-A2 0 121 877 können eingesetzt werden. Die Zerstäubungsenergie wird hierbei durch den Druck einer Pumpe erzeugt, die die zu trocknende Flüssigkeit der Einstoffhohlkegeldüse zuleitet. Der für die Zerstäubung erforderliche Druck kann bis zu 80 bar absolut betragen, vorzugsweise werden jedoch nur Drucke von 15 bis 25 bar absolut angewendet. Der in der zu trocknenden Flüssigkeit enthaltende Feststoffanteil kann bis zu 45 Gew.%, vorzugsweise 15 bis 30 Gew.% betragen.

Die Riboflavinsuspension wird mittels der beschriebenen Einstoffhohlkegeldüse in einen beheizten Trockenturm eingetragen, hier getrocknet und am unteren Ende des Turms ausgetragen. Die Trockenturmeingangstemperatur des Trocknungsgases beträgt im allgemeinen etwa 100 bis 200, vorzugsweise 130 bis 170°C, die Verweilzeit beträgt im allgemeinen etwa 20 bis 40 Sekunden. Um eine nicht staubende Vitamin-B₂-Mikrogranulatfraktion zu erhalten, muß die im Trockenturm anfallende Trockenware einer geeigneten Trennvorrichtung zugeführt werden. Als Trennvorrichtung kann der Trockenturm-Austragskonus ausgebildet werden, wie dies beispielsweise in der Patentschrift DE 33 44 509 beschrieben ist. In dieser Trennvorrichtung wird das nicht staubende Mikrogranulat als Nutzfraktion abgeschieden, während die kleineren Partikel (< 20 µm) den Trockenturm mit dem Trocknungsgas verlassen. Dieses Feingut wird in nachgeordneten Abscheidern (Zyklone, Filter) vom Gasstrom abgetrennt und kann dem zu trocknenden wäßrigen Riboflavinsuspensionen zugemischt werden (Rückführung). Der Anteil der staubenden Feinfraktion ist abhängig vom Feststoffgehalt des der Hohlkegeldüse zugeleiteten Suspension sowie vom Düsenvordruck. Der Feinanteil kann ca. 5 bis 40 % betragen. Bei einem Feststoffgehalt des Austrags von ca. 25 bis 30 % und einem Düsenvordruck von ca. 15 bar beträgt der rückzuführende Feinanteil nur ca. 5 bis 10 %.

Das auf diese Weise erhaltene Produkt weist einen Partikelgrößenbereich von 30 bis 200 µm auf.

Auch das so erhaltene Mikrogranulat zeichnet sich durch hervorragende Handlingseigenschaften aus.

Auch durch Scheibenzerstäubungstrocknung von Riboflavinsuspensionen kann man auch ohne Zusatz von nennenswerten Mengen an Bindemitteln ein Mikrogranulat herstellen, welches nach der Abtrennung des Feingutanteils (< 20 µm) wie sie oben beschrieben wurde, sehr gute Handlingseigenschaften aufweist.

Die nach dem erfindungsgemäßen Verfahren auf technisch einfache Weise hergestellten Riboflavinsprüh- oder -mikrogranulate besitzen überraschenderweise gegenüber den bislang bekannten und handelsüblichen Riboflavinpräparaten erhebliche anwendungstechnische Vorteile. Besondere Vorteile bieten sie in Anwendungsbereichen, in denen die Anwesenheit von Bindemitteln oder Granulierhilfsmitteln unerwünscht ist.

### Beispiel 1

In einem Wirbelschichttrockner wurden etwa 2,5 kg/h einer ca. 20 %igen wäßrigen Suspension eines sehr feinteiligen Riboflavins (Schüttgewicht ca. 0,1 kg/l; Riboflavingehalt 99,5 %; Pharmaware) von 20°C kontinuierlich mittels einer Zweistoffdüse in eine Wirbelschicht aus Riboflavin etwa gleicher Zusammensetzung eingesprüht. Die Wirbelgaseintrittstemperatur betrug 170°C. Die Einspritzmenge wurde so bemessen, daß sich eine Wirbelbettemperatur von 71 bis 72°C einstellte.

Ein Teil der Wirbelbettvorlage wurde kontinuierlich abgezogen und durch Siebeinrichtungen kontinuierlich in 3 Partikelfraktionen getrennt. Das erhaltene Trockenprodukt wies folgende Partikelgrößenverteilung auf:
1) 20 bis 30 % eines Produktes mit einem Partikelgrößenbereich < 100 µm
2) 30 bis 40 % des Wertproduktes mit einem Partikelgrößenbereich von 100 bis 300 µm
3) 30 bis 50 % eines Produktes mit einem Partikelgrößenbereich > 300 µm.

Die Grobfraktion 3) wurde gemahlen bis zu einem Partikelgroßenbereich von < 250 µm und dann zusammen mit der feinteiligen Fraktion 1) kontinuierlich wieder in die Wirbelschicht zurückgeführt.

Etwa 0,5 kg/h an dem gewünschten Riboflavinsprühgranulat (Partikelgrößenbereich 125 bis 250 µm) wurden als Wertprodukt erhalten.

### Beispiel 2

In einem Wirbelschichttrockner wurden 2,5 kg/h einer ca. 20 %igen wäßrigen Suspension eines sehr feinteiligen handelsüblichen Riboflavins (Schüttgewicht ca. 0,1 kg/l; Riboflavingehalt 96 %; Futtermittelqualität) in ein Riboflavinwirbelbett eingesprüht. Das Wirbelgas hatte eine Gaseintrittstemperatur von 160 bis 170°C. Die Einspritzmenge wurde so bemessen, daß sich eine Wirbelschichttemperatur von 78 bis 80°C einstellte.

Analog Beispiel 1 wurden etwa 0,5 kg/h an Riboflavinsprühgranulat mit dem gewünschten Partikelgrößenbereich von 125 bis 250 µm erhalten.

## Patentansprüche

1. Verfahren zur Herstellung von rieselfähigen, nichtstaubenden bindmittelfreien Riboflavinsprühgranulaten oder -mikrogranulaten mit einem Partikelgrößenbereich von 50 bis 450 µm aus feinteiligem reinem Riboflavin, dadurch gekennzeichnet, daß man eine wäßrige oder mindestens 10 Gew.-% Wasser enthaltende Suspension, enthaltend etwa 5 bis 30 Gew.-% an reinem feinteiligem Riboflavin
a) einer Sprühwirbelschichttrocknung
b) einer Einstoffdüsenzerstäubungstrocknung oder
c) einer Scheibenzerstäubungstrocknung
bei Temperaturen von 20 bis 100°C zuführt, ohne daß der Suspension Bindemittel zugesetzt werden.

2. Verfahren zur Herstellung von rieselfähigen, nicht staubenden bindemittelfreien Riboflavinsprühgranulaten aus feinteiligem reinem Riboflavin gemäß Anspruch 1, dadurch gekennzeichnet, daß man eine wäßrige oder Wasser enthaltende Suspension des reinen feinteiligen Riboflavins einer Sprühwirbelschichttrocknung zuführt, ohne daß der Suspension Bindemittel zugesetzt werden.

3. Verfahren gemäß Anspruch 2, dadurch gekennzeichnet, daß man eine wäßrige Suspension des reinen feinteiligen Riboflavins der Sprühwirbelschichttrocknung zuführt.

4. Verfahren gemäß Anspruch 2, dadurch gekennzeichnet, daß man zur Durchführung der Sprühwirbelschichttrocknung
a) Riboflavin in Form eines Riboflavintrockenpulvers oder eines Sprüh-oder Mikrogranulates in einem Wirbelschichttrockner in einem auf 20 bis 100°C gehaltenen Wirbelbett vorlegt,
b) hierzu die wäßrige oder Wasser enthaltende Suspension des reinen feinteiligen Riboflavins nach Maßgabe der Trocknungsgeschwindigkeit in versprühter Form zugibt,
c) die Riboflavinpartikel nach geeigneter Verweilzeit aus dem Wirbelbett abzieht und durch eine geeignete Vorrichtung in Partikelfraktionen auftrennt,
d) die Partikelfraktion im Partikelgrößenbereich von etwa 50 bis 450 µm ausschleust und
e) die feinerteiligen Partikel und/oder die durch Vermahlen von größeren Partikeln erhaltenen feinteiligen Partikel in den Granulierungsprozeß zurückführt.

5. Verfahren gemäß Anspruch 4, dadurch gekennzeichnet, daß man zur kontinuierlichen Durchführung der Sprühwirbelschichttrocknung ein auf 50 bis 90°C gehaltenes Wirbelbett, bestehend aus Riboflavinsprüh- oder -mikrogranulaten verwendet, kontinuierlich einen Teil des erhaltenen getrockneten Produktes aus der Wirbelschichtvorlage entnimmt und in Partikelfraktionen auftrennt, die Partikelfraktion im Partikelgrößenbereich von etwa 100 bis 450 µm als Wertprodukt ausschleust und die feinerteiligen Partikel und/oder die durch Vermahlen von größeren Partikeln erhaltenen feinerteiligen Partikel in das Wirbelbett zurückführt um den Granulationsprozeß aufrecht zu erhalten.

6. Verfahren gemäß Anspruch 4, dadurch gekennzeichnet, daß man ein auf 60 bis 80°C gehaltenes Wirbelbett, bestehend aus Riboflavinsprüh- oder -mikrogranulaten, verwendet.

7. Verfahren gemäß Anspruch 1 zur Herstellung von Mikrogranulaten, dadurch gekennzeichnet, daß man zur Durchführung der Einstoffdüsenzerstäubungstrocknung die wäßrige oder Wasser enthaltende Suspension des feinteiligen Riboflavins unter einem Druck von 5 bis 80 bar (absolut) durch eine Einstoffhohlkegeldüse in einen geeigneten Trockenturm einsprüht.

## Claims

1. A process for preparing spray granules or micro-granules, which contain no binder, are non-dusting and free-flowing and have a particle size range from 50 to 450 µm, from finely divided pure riboflavin, which comprises subjecting an aqueous suspension or a suspension containing at least 10% by weight of water, which contains from about 5 to 30% by weight of pure finely divided riboflavin, to
a) a fluidized bed spray drying
b) a single-fluid nozzle spray drying or
c) a disk-type spray drying
at from 20 to 100°C without adding binders to the suspension.

2. A process for preparing spray granules, which contain no binder, are non-dusting and free-flowing, from finely divided pure riboflavin and claimed in claim 1, which comprises subjecting an aqueous or water-containing suspension of the pure finely divided riboflavin to a fluidized bed spray drying without adding binders to the suspension.

3. A process as claimed in claim 2, wherein an aqueous suspension of the pure finely divided riboflavin is subjected to the fluidized bed spray drying.

4. A process as claimed in claim 2, wherein the fluidized bed spray drying is carried out by
a) introducing riboflavin in the form of a dry powder or of spray granules or microgranules into a fluidized bed drier in which the bed is kept at from 20 to 100°C,
b) adding to this the aqueous or water-containing suspension of the pure finely divided riboflavin in sprayed form in accordance with the rate of drying,
c) after a suitable residence time, drawing off the riboflavin particles from the fluidized bed and separating them into fractions in a suitable apparatus,
d) ejecting the fraction with the particle size range from about 50 to 450 µm and
e) returning the finer particles and/or the fine particles obtained by milling larger particles to the granulation process.

5. A process as claimed in claim 4, wherein the fluidized bed spray drying is carried out continuously by using a fluidized bed which is composed of spray granules or microgranules of riboflavin and is kept at from 50 to 90°C, continuously removing a portion of the resulting dry product from the fluidized bed and separating it into particle fractions, ejecting the fraction in the particle size range from about 100 to 450 µm as required product, and returning the finer particles and/or the finer particles obtained by milling larger particles to the fluidized bed in order to maintain the granulation process.

6. A process as claimed in claim 4, wherein a fluidized bed which is composed of spray granules or micro-granules of riboflavin and is kept at from 60 to 80°C is used.

7. A process as claimed in claim 1 for preparing microgranules, wherein the single-fluid nozzle spray drying is carried out by spraying the aqueous or water-containing suspension of the finely divided riboflavin under a pressure of from 5 to 80 bar (absolute) through a single-fluid hollow-cone nozzle into a suitable drying tower.

## Revendications

1. Procédé de fabrication de granulés ou microgranulés de pulvérisation de riboflavine exempts de liant, ne dégageant pas de poussière et fluides, d'une plage de tailles de particules allant de 50 à 450 µm, à partir de riboflavine pure finement divisée, caractérisé en ce que l'on soumet une suspension aqueuse ou contenant au moins 10 % en pour-cent en poids d'eau, et contenant environ 5 à 30 % en poids de riboflavine pure,
a) à un séchage en couche tourbillonnaire, par pulvérisation
b) à un séchage par pulvérisation à buses monoproduit ou
c) à un séchage par pulvérisation à disques
à des températures de 20 à 100°C, sans ajouter de liant à la suspension.

2. Procédé de fabrication de granulats de pulvérisation de riboflavine exempts de liant ne produisant pas de poussière, fluides, à partir de riboflavine pure, finement divisée selon la revendication 1, caractérisé en ce que l'on soumet une suspension, aqueuse ou contenant de l'eau, de la riboflavine pure, finement divisée, à un séchage en couche tourbillonnaire par pulvérisation, sans ajouter de liant à la suspension.

3. Procédé selon la revendication 2, caractérisé en ce qu'on amène une suspension aqueuse de riboflavine pure, finement divisée, au séchage à couche tourbillonnaire par pulvérisation.

4. Procédé selon la revendication 2, caractérisé en ce que pour effectuer le séchage en couche tourbillonnaire par pulvérisation
a) on part de riboflavine se présentant sous la forme d'une poudre sèche de riboflavine ou d'un granulé de pulvérisation ou microgranulé, que l'on introduit dans un séchoir à couche tourbillonnaire, à l'intérieur d'une couche tourbillonnaire maintenue à une temperature de 20 à 100°C,
b) on y ajoute sous forme pulvérisée la suspension aqueuse ou contenant de l'eau de riboflavine finement divisée d'après la valeur de la vitesse de séchage,
c) on effectue la séparation d'une couche tourbillonnaire des particules de riboflavine après écoulement d'un temps de séjour approprié, et l'on effectue le tri des fractions de particules dans un dispositif approprié,
d) on évacue la fraction de particules située dans la zone de taille de particules d'environ 50 à 450 µm, et
e) on recycle dans le processus de granulation les particules finement divisées et/ou les particules finement divisées obtenues par broyage des particules plus grosses.

5. Procédé selon la revendication 4, caractérisé en ce que pour effectuer en continu le séchage en couche tourbillonnaire par pulvérisation, on utilise une couche tourbillonnaire maintenue à une température de 50 à 90°C constitué de granulés de pulvérisation de riboflavine ou de microgranulés de riboflavine, on effectue un prélèvement continu d'une partie séchée obtenue pour l'extraire de la masse de la couche tourbillonnaire et on effectue un tri selon les fractions de particules, on évacue la fraction de particules située dans la plage de tailles de particules allant d'environ 100 à 450 µm à titre de produit ayant la valeur souhaitée et l'on recycle les particules finement divisées et/ou les particules finement divisée obtenues par broyage de particules plus grosses en les réintroduisant dans la couche tourbillonnaire afin d'obtenir le maintien en fonctionnement du processus de granulation.

6. Procédé selon la revendication 4, caractérisé en ce qu'on utilise une couche tourbillonnaire maintenue à une température de 60 à 80°C, constitué de granulés de pulvérisation de riboflavine ou de microgranulés de riboflavine.

7. Procédé selon la revendication 1 pour la fabrication de microgranulés, caractérisé en ce que pour effectuer le séchage par pulvérisation à buse monoproduit on introduit avec pulvérisation la suspension aqueuse ou contenant de l'eau de la riboflavine finement divisée, ceci sous une pression de 5 à 80 bar (pression absolue) dans une buse conique creuse monoproduit, dans une tour de séchage appropriée.
